# EUROPEAN PATENT APPLICATION

(11) **EP 4 438 113 A1**
(43) Date of publication of application: **02.10.2024**
(21) Application number: 22919052.5
(22) Date of filing: 16.12.2022
(51) Int. Cl.: A61N 7/02, A61N 7/00

(54) **STRUCTURE FOR MAXIMIZING OUTPUT OF VIBRATION WAVE OF TRANSDUCER HOLDER OF HIGH-INTENSITY FOCUSED ULTRASOUND GENERATION DEVICE**

(30) Priority: 05.01.2022 KR 20220001327
(71) Applicant: Jeisys Medical Inc., Geumcheon-gu Seoul 08501 (KR)
(72) Inventor: YI, Won Ju, Seoul 08501 (KR); GONG, Eun Kyung, Seoul 08501 (KR); HWANG, Soo Min, Seoul 08501 (KR); KANG, Dong Hwan, Seoul 08501 (KR)
(74) Representative: dompatent von Kreisler Selting Werner - Partnerschaft von Patent- und Rechtsanwälten mbB
(86) International application number: PCT/KR2022/020528
(87) International publication number: WO 2023/132515

(57) **Abstract**

In a high-intensity focused ultrasound generation device according to the present invention, each of a plurality of transducers is mounted to a transducer holder, the transducer holder is detachably coupled to an ultrasonic radiation frame, thereby facilitating the individual replacement and repair of each transducer, in addition, at least a portion of a side surface of the transducer holder is incised to form an opening, and thus the vibration wave energy radiating forward from the transducers seated on the transducer holders is maximized, thereby improving output. In addition, the transducer is seated on support protrusions of the transducer holder, and the transducer holder and the transducer are bonded with flexible glue, so that the position of the transducer is fixed while the transducer is capable of vibrating, thereby reducing vibration wave energy loss of the transducer.

## Description

### Technical field

The present invention relates to a structure for maximizing output of a vibration wave of a transducer holder of a high-intensity focused ultrasound generation device, and more particularly, to a structure for maximizing output of a vibration wave of a transducer holder of a high-intensity focused ultrasound generation device, whereby a plurality of transducers are individually mounted on an ultrasonic radiation frame using transducer holders and vibration wave energy of the plurality of transducers mounted on the transducer holders can be maximized.

### Background art

In general, high-intensity focused ultrasound generation devices are apparatuses that generate high-intensity ultrasonic energy by focusing ultrasonic waves generated by transducers to rise the temperature of an affected area by irradiating high-intensity ultrasonic energy to the affected area of a patient so that the affected area can be treated without surgery.

When several tens or several hundreds of transducers are used in a high-intensity focused ultrasound generation device according to the related art, a plurality of transducers are mounted on the front surface of an ultrasonic radiation frame and then the whole front surface of the ultrasonic radiation frame is coated with glue to form a waterproof layer so that the plurality of transducers are fixed by the waterproof layer and a leakage can be prevented.

However, since ultrasonic energy generated by the transducers in a forward direction is absorbed by the waterproof layer, there is a problem that input voltages need to be increased to compensate therefor, and there is a problem that the ultrasonic radiation frame needs to be replaced even if only one of the plurality of transducers breaks down.

### Detailed description of the invention

### Technical problem

The present invention provides a structure for maximizing output of a vibration wave of a transducer holder of a high-intensity focused ultrasound generation device, whereby vibration wave energy of a transducer can be maximized and thus output can be enhanced.

### Technical solution

According to an aspect of the present invention, there is provided a structure for maximizing output of a vibration wave of a transducer holder of a high-intensity focused ultrasound generation device, the structure including: an ultrasonic radiation frame having a concave front surface and a plurality of coupling holes formed therein; a plurality of transducer holders respectively inserted into the plurality of coupling holes in front of the ultrasonic radiation frame and detachably coupled to the ultrasonic radiation frame while passing through the ultrasonic radiation frame; and a plurality of transducers respectively mounted in the plurality of transducer holders so that front surfaces of the plurality of transducers are exposed, wherein at least a part of a side surface of the transducer holder is incised to form an opening so as to maximize vibration wave energy generated in the transducer and radiating therefrom.

A flexible glue layer filled with flexible glue may be formed between the transducer and the transducer holder.

Each of the plurality of transducer holders may include: a head portion mounted on the front surface of the ultrasonic radiation frame, having a seating groove in which the transducer is insertedly seated, formed therein having the opening formed therein; and a body portion extending from the head portion backward and coupled to the coupling holes using a fastening member from a rear of the ultrasonic radiation frame while passing through the coupling holes.

At least one support protrusion may be protrudedly formed on a side surface of the seating groove to support a lower surface of the transducer and to form a separation space between the transducer and a bottom surface of the seating groove.

At least one support protrusion may be protrudedly formed on the bottom surface of the seating groove to support a lower surface of the transducer and to form a separation space between the transducer and a bottom surface of the seating groove.

A flexible glue layer filled with flexible glue may be formed between the lower surface of the transducer and an upper surface of the support protrusion to reduce loss of vibration wave energy of the transducer.

The body portion may include supply holes formed in a longitudinal direction so that a power supply unit coupled to the transducer passes through the power supply holes.

A glue layer for waterproofing filled with glue for waterproofing may be formed between the power supply unit and the power supply holes.\

The body portion may include: a shaft portion extending from the head portion backward and being pressed into the coupling holes; and a screw portion extending from the shaft portion backward and coupled to the fastening member in the rear of the ultrasonic radiation frame after passing through the coupling holes.

According to another aspect of the present invention, there is provided a structure for maximizing output of a vibration wave of a transducer holder of a high-intensity focused ultrasound generation device, the structure including: an ultrasonic radiation frame having a concave front surface and a plurality of coupling holes formed therein; a plurality of transducer holders respectively inserted into the plurality of coupling holes in front of the ultrasonic radiation frame and detachably coupled to the ultrasonic radiation frame while passing through the ultrasonic radiation frame; and a plurality of transducers respectively mounted in the plurality of transducer holders so that front surfaces of the plurality of transducers are exposed, wherein the transducer holder includes: a head portion mounted on the front surface of the ultrasonic radiation frame and having a seating groove in which the transducer is insertedly seated, formed therein; and a body portion extending from the head portion backward and coupled to the coupling holes using a fastening member from a rear of the ultrasonic radiation frame while passing through the coupling holes, and at least a part of a side surface of the head portion is incised to form an opening so as to maximize vibration wave energy generated in the transducer, and a support protrusion is protrudedly formed on a bottom surface of the seating groove to support a lower surface of the transducer and to form a separation space between the transducer and the bottom surface, and a flexible glue layer filled with flexible glue is formed between the transducer and the support protrusion.

### Effects of the invention

In a high-intensity focused ultrasound generation device according to the present invention, a plurality of transducers are individually mounted on transducer holders, and the transducer holders are detachably coupled to an ultrasonic radiation frame and thus, transducers can be easily individually replaced or repaired, and in addition, at least a portion of a side surface of the transducer holder is incised to from an opening, and thus the vibration wave energy radiating forward from the transducers seated on the transducer holders is maximized and thus, output can be enhanced.

In addition, the transducer is seated on support protrusions of the transducer holder, and adhesion between the transducer holder and the transducer is performed with flexible glue so that the position of the transducer is fixed while vibration of the transducer is enabled, and thus, the loss of vibration wave energy of the transducer can be reduced.

### Description of the drawings

FIG. 1 is a perspective view illustrating a head module of a high-intensity focused ultrasound generation device according to an embodiment of the present invention.
FIG. 2 is an exploded perspective view illustrating a coupling structure of an ultrasonic radiation frame and a transducer holder according to an embodiment of the present invention.
FIG. 3 is an enlarged view of a portion A of FIG. 1;
FIG. 4 is a cross-sectional view illustrating a coupling state of a transducer and a transducer holder according to an embodiment of the present invention.
FIG. 5 is a front perspective view of a transducer holder according to an embodiment of the present invention.
FIG. 6 is a rear perspective view of the transducer holder shown in FIG. 5.
FIG. 7 is a front perspective view of a transducer holder according to another embodiment of the present invention.

### Mode of the invention

Hereinafter, embodiments of the present invention will be described in detail with reference to the accompanying drawings.

A high-intensity focused ultrasound generation device according to an embodiment of the present invention is an apparatus using high-intensity focused ultrasound (HIFU). The high-intensity focused ultrasound generation device may include a transducer array in which several tens or several hundreds of transducers are radially arranged, thereby treating the affected area of a patient with tumor, etc., stimulating the brain to treat diseases such as Alzheimer's or depression, and increasing immunity by heating specific areas.

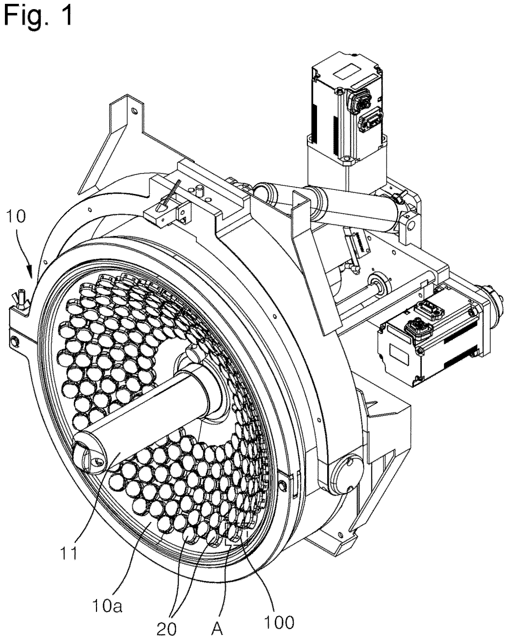

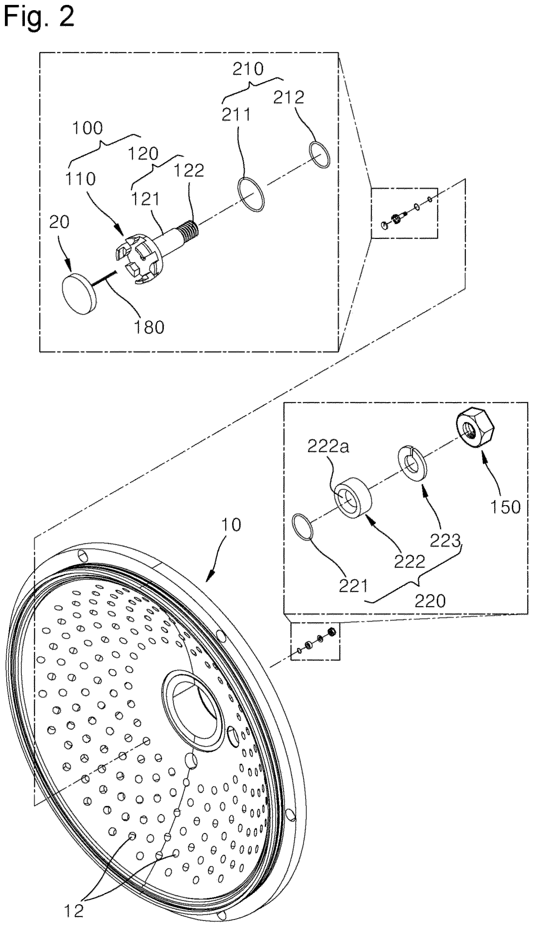

FIG. 1 is a perspective view illustrating a head module of a high-intensity focused ultrasound generation device according to an embodiment of the present invention. FIG. 2 is an exploded perspective view illustrating a coupling structure of an ultrasonic radiation frame and a transducer holder according to an embodiment of the present invention.

Referring to FIGS. 1 and 2, the head module of the high-intensity focused ultrasound generation device includes an ultrasonic radiation frame 10, a plurality of transducers 20, and a plurality of transducer holders 100.

The ultrasonic radiation frame 10 includes a probe 11 coupled to the center of a front surface 10a, and a plurality of coupling holes 12 arranged radially based on the probe 11. The ultrasonic radiation frame 10 is formed to have a dish shape with a concave front surface to focus ultrasound waves radiating from the plurality of transducers 20 to radiate the ultrasound waves to one place.

The plurality of coupling holes 12 are through holes spaced apart from each other at certain intervals. The number of the coupling holes 12 is set according to the number of the transducers 20.

The plurality of transducers 20 may include a piezoelectrical element. Each of the transducers 20 generates ultrasound waves when receiving voltages. An example in which the transducer 20 has a disc shape, will be described. Several tens or several hundreds of transducers 20 are radially arranged to form a transducer array. The number of the transducers 20 may be set according to ultrasonic energy to radiate.

A power supply unit is connected to upper and lower ends of the transducer 20. An example in which the power supply unit is first and second electrode wires 21 and 22, will be described. The first electrode wire 21 is connected to the transducer 20 at the center of the front surface of the transducer 20 by soldering, and the second electrode wire 22 is connected to the transducer 20 at the center of a rear surface of the transducer 20 by soldering. The first electrode wire 21 and the second electrode wire 22 are pulled out to the rear of the ultrasonic radiation frame 10 through electrode wire holes of the transducer holder 100 and are connected to a separate circuit board. However, the present invention is not limited thereto, and the power supply unit capable of supplying power such as a pin, a connector or the like, may be applied.

The transducer holder 100 is detachably coupled to each of the plurality of coupling holes 12. Each transducer 20 is coupled to the transducer holder 100.

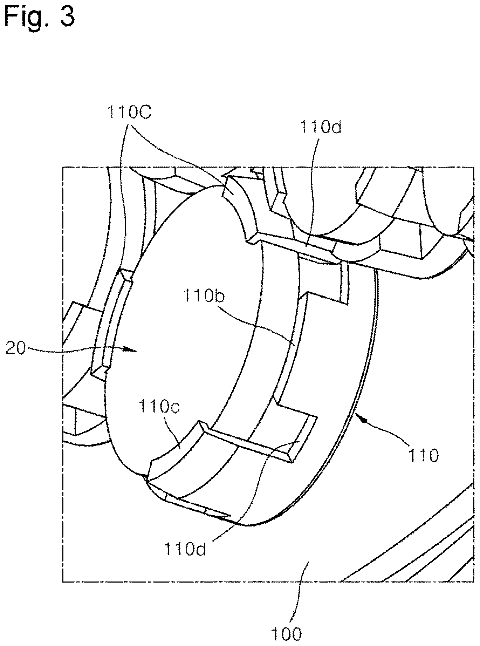

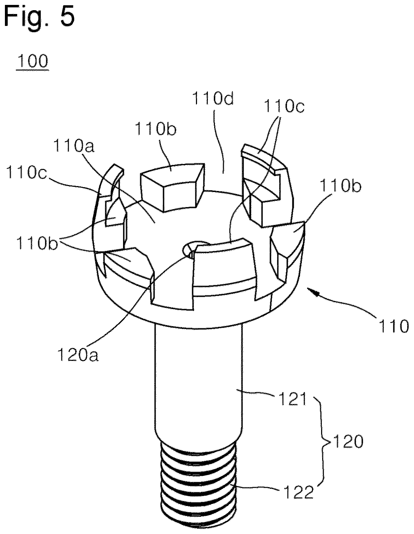

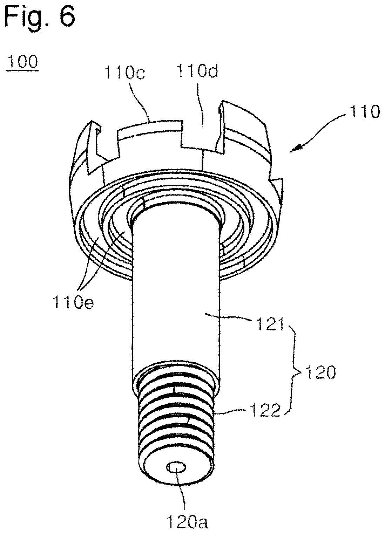

Referring to FIGS. 3 through 6, the transducer holder 100 includes a head portion 110 with a seating groove 110a into which the transducer 20 is insertedly seated, and a body portion 120 which extends from the head portion 110 backward and is coupled to the coupling holes 12.

The head portion 110 is greater in diameter than the coupling holes 12 so as to be seated on the front surface 10a of the ultrasonic radiation frame 10. The head portion 110 includes the seating groove 110a, a support protrusion 110b, a locking protrusion 110c, and an opening 110d.

The seating groove 110a is formed in the front of the head portion 110 to have an open front surface, such that the transducer 20 is seated thereon.

The support protrusion 110b is a stepped protrusion that protrudes from a bottom surface of the seating groove 110a in a forward direction to a certain height so as to support a lower surface of the transducer 20. The support protrusion 110b forms a separation space S between the lower surface of the transducer 20 and the bottom surface of the seating groove 110a to form a passage through which the first and second electrical wires 21 and 21 coupled to the transducer 20 pass, thereby implementing an electrode structure stably and preventing the loss of the vibration wave energy of the transducer 20 by enabling vibration of the transducer 20. An example in which the support protrusion 110b is formed in plurality and a plurality of support protrusions 110b are spaced apart from each other at certain intervals, will be described. Also, the support protrusion 110b may also be formed as one body with the head portion 110 and may also be formed by applying a non-conductive material, for example, glue onto the bottom surface of the seating groove 110a.

The locking protrusion 110c protrudes from the bottom surface of the seating groove 110a and has a tip bent inwardly so that deviation of the transducer 20 inserted into the seating groove 110a can be prevented. The tip of the locking protrusion 110c may be changed as long as it has a shape in which deviation of the transducer 20 can be prevented, such as a hook shape, etc. The locking protrusion 110c is formed in plurality, and the plurality of locking protrusions 110c are spaced apart from each other at certain intervals. In the present embodiment, an example in which some of the plurality of locking protrusions 110c protrude from the support protrusions 11 0b, will be described.

The opening 110d is cut from side surfaces of the seating groove 110a and is formed to be open. There is an advantage in that assembling can be easily performed by the opening 110d. In addition, the opening 110d allows a vibration wave energy that is generated in the transducer 20 and radiating forward, to be maximized.

It is preferable that the body portion 120 is formed to extend from the head portion 110 backward and to pass through the coupling holes 12. The body portion 120 is formed to have a smaller diameter than a diameter of the head portion 110. Power supply holes are formed in the center of the body portion 120 so that the power supply unit may pass through the power supply holes. An example in which the power supply holes are electrode wire holes 120a through which the first and second electrode wires 21 and 22 pass, will be described.

The body portion 120 includes a shaft portion 121 and a screw portion 122.

The shaft portion 121 extends from the head portion 110 backward and is formed in a cylindrical shape to be pressed into the coupling holes 12.

The screw portion 122 extends from the shaft portion 121 backward, and has a screw thread fastened to an outer circumferential surface of the screw portion 122 by a fastening member 150.

The fastening member 150 is preferably, a nut, but embodiments are not limited thereto.

The electrode wire holes 120a are formed in the center of the body portion 120 so that the first and second electrode wires 21 and 22 may be inserted into and may pass through the electrode wire holes 120a.

A glue layer 250 for waterproofing is formed between the electrode wire holes 120a and the first and second electrode wires 21 and 22. The glue for waterproofing may be silicon or epoxy-based glue, and may be applied as long as it is formed of a non-conductive adhesive material. The glue for waterproofing may be the same as flexible glue to be described below. In the present embodiment, an example in which the glue layer 250 for waterproofing is provided only at an upper side of the electrode wire holes 120, but the glue layer 250 for waterproofing may also be formed to fill the whole separation space S with the glue for waterproofing.

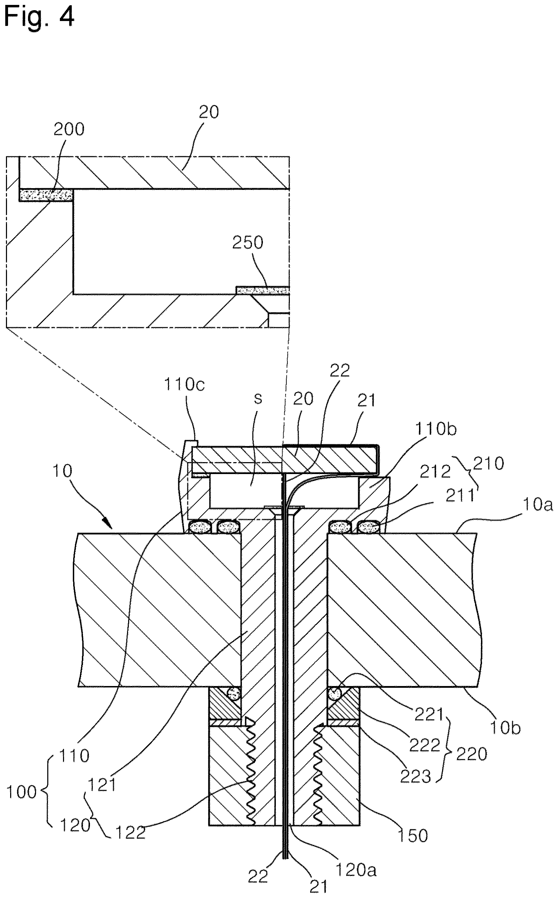

Referring to FIG. 4, a flexible glue layer 200 is formed between at least one of the rear surface and the side surface of the transducer 20 and the transducer holder 30.

The flexible glue layer 200 is a layer filled with an adhesive member such as flexible glue or the like. The flexible glue may be silicon or epoxy-based glue, and may be applied as long as it is formed of a flexible and non-conductive adhesive material.

In the present embodiment, an example in which the flexible glue layer 200 is formed between the rear surface of the transducer 20 and the support protrusion 110b, will be described. However, embodiments are not limited thereto, and the flexible glue layer 200 may be formed between the side surface of the transducer 20 and an inner side of each of the locking protrusions 110c. That is, the flexible glue layer 200 may be applied in any location where the front surface of the transducer 20 is not blocked. Also, the flexible glue layer 200 may also be formed to fill the whole separation space S with the flexible glue.

The transducer 20 is adhered to the transducer holder 100 and is fixed thereto using the flexible glue, so that the location of the transducer 20 is fixed inside the transducer holder 100 and the transducer 20 may vibrate and thus, the vibration wave energy loss of the transducer 20 may be minimized. Also, since the glue is not applied onto the front surface of the transducer 20, the loss of ultrasonic energy radiating from the transducer 20 forward can be prevented. That is, since the flexible glue layer 200 is formed at only the rear surface or side surface of the transducer 20, the flexible glue layer 200 does not cover the front surface of the transducer 20 and thus, there are no restrictions in radiation of ultrasonic energy through the front surface.

Meanwhile, sealing between the transducer holder 100 and the ultrasonic radiation frame 10 is performed using a sealing member.

The sealing member includes a first sealing member 210 for sealing between the head portion 110 of the transducer holder 100 and the front surface 10a of the ultrasonic radiation frame 10, and a second sealing member 220 for sealing between the body portion 120 and a rear surface 10b of the ultrasonic radiation frame 10.

An example in which the first sealing member 210 includes two first and second O-rings 211 and 212 that are inserted into the rear surface of the head portion 110 and coupled to each other, will be described. However, embodiments are not limited thereto, and the number of the first sealing members 210 may be changed in various ways and applied. Also, the first sealing member 210 may be used as long as it is formed of various materials such as silicon, rubber or the like and has a sealing structure, except for an O-ring.

It is preferable that the first O-ring 211 and the second O-ring 212 are formed to have different diameters. The first O-ring 211 and the second O-ring 212 are inserted into a ring-shaped groove 110e formed in the rear surface of the head portion 110 and are sealed while being in close contact with the front surface 10a of the ultrasonic radiation frame 10.

The second sealing member 220 includes a third O-ring 221 inserted outside the shaft portion 121 of the body portion 120, and an O-ring pressing member 222 inserted outside the shaft portion 121 in a backward direction of the third O-ring 221 and allowing the third O-ring 221 to be in close contact with the rear surface 10b of the ultrasonic radiation frame 10.

The O-ring pressing member 222 is formed to have a ring shape, and includes an inclined surface 222a on which a part of the third O-ring 221 is seated and which is formed on the front surface of the O-ring pressing member 222.

The second sealing member 220 may further include a washer 223 provided between the O-ring pressing member 222 and the fastening member 150. The washer 223 is not an essential component of the second sealing member 220 and may be additionally provided. The washer 223 may seal the third O-ring 221 and the O-ring pressing member 222 and may hold the transducer holder 100.

The second sealing member 220 may be used as long as it is formed of various materials such as silicon, rubber, etc. and seals, except for an O-ring or a washer.

Meanwhile, in the present embodiment, an example in which the first and second electrode wires 21 and 22 are coupled to the transducer 20, will be described, but embodiments are not limited thereto, and the transducer holder 100 may be coated with a conductive material to form an electrode, and the front surface and the side surface of the transducer are in contact with the electrode and are grounded, and a power supply unit such as an electrode wire may also be coupled to the rear surface of the transducer.

In the high-intensity focused ultrasound generation device having the above-described configuration, the plurality of transducers 20 are mounted on the ultrasonic radiation frame 10 using the transducer holder 100, so that adhesion between the transducer 20 and the transducer holder 100 is performed using the flexible glue and sealing therebetween is performed, even if the glue is not applied onto all of the front surface of the ultrasonic radiation frame 10, a leakage can be prevented from occurring inwardly from the front surface of the ultrasonic radiation frame 10.

Also, at least a part of a side surface of the transducer holder 200 is incised to form an opening 110d so that vibration wave energy radiating from the transducer 20 seated on the transducer holder 200 forward may be maximized and thus, output may be enhanced.

In addition, since the glue is not applied onto all of the front surface of the ultrasonic radiation frame 10, all of front surfaces of the transducers 20 is exposed so that the loss of ultrasonic energy radiating from the transducers 20 forward can be prevented. When, as in the related art, the front surfaces of the transducers 20 are blocked by the glue layer, there is a problem that ultrasonic energy is absorbed by the glue layer, but in the present invention, all of the front surfaces of the transducers 20 is exposed and thus this problem can be prevented.

Further, adhesion between the inside of the transducer holder 100 and the transducer 20 is performed using the flexible glue so that the location of the transducer 20 is fixed and vibration of the transducer 20 is enabled while a gap is prevented and thus, the loss of vibration wave energy of the transducer 20 can be reduced.

Moreover, the plurality of transducers 20 are individually mounted through the transducer holders 100, and the transducer holders 100 are detachably coupled to the ultrasonic radiation frame 10 and thus, repair and replacement of the transducers 20 may be individually performed.

In addition, since the plurality of transducers 20 are individually mounted through the transducer holder 100, the capacity of at least a part of the plurality of transducers 20 may be differently configured. For example, the capacity of transducers arranged at the center of the ultrasonic radiation frame 10 may also be increased, and of course, voltages applied to the plurality of transducers 20 may also be differently controlled.

Also, sealing between the transducer holder 100 and the ultrasonic radiation frame 10 may be performed using a sealing member such as an O-ring, so that a leakage can be prevented from occurring from the front surface of the ultrasonic radiation frame 10 backward and the transducer holders may be easily attached to/detached from the ultrasonic radiation frame.

Meanwhile, in the above-described embodiment, an example in which all of the transducers 20 are coupled to the coupling holes 12 of the ultrasonic radiation frame 10, has been described, but embodiments are not limited thereto, and the transducer 20 may also be provided in only at least a part of the coupling holes 12 according to the capacity of the high-intensity focused ultrasound generation device. When the transducers 20 are provided in only at least a part of the coupling holes 12, the transducer holders 100 are coupled to all of the coupling holes 12, and a holder cover (not shown) for shielding the open front surface may also be detachably coupled to some of the transducer holders 100 to which the transducers 20 are not coupled. The holder cover (not shown) may be formed of a different material from the transducers 20, may have the same shape as the transducers 20 and may be coupled to the transducers 20 using glue. Thus, the mounting number of the transducers 20 can be adjusted so that the energy capacity of the high-intensity focused ultrasound generation device can be adjusted.

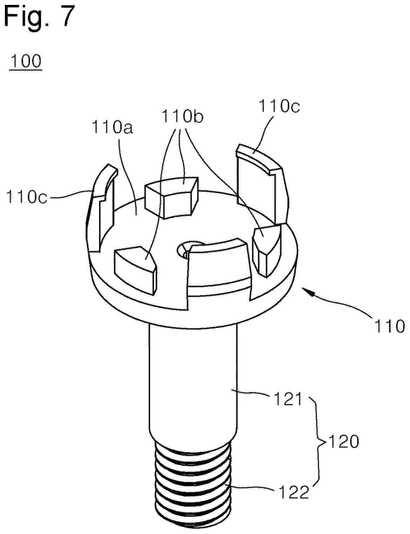

Meanwhile, FIG. 7 is a front perspective view of a transducer holder according to another embodiment of the present invention.

Referring to FIG. 7, support protrusions 110b of the transducer holder provided on the bottom surface of the seating groove 110a are different from the above-described embodiment, and the other configuration and operation of FIG. 7 are similar to those of the above-described embodiment and thus, hereinafter, differences therebetween will be mainly described.

An example in which the support protrusions 110b protrude from the bottom surface of the seating groove 110a and three support protrusions are provided to be spaced apart from each other by a certain distance, has been described. However, embodiments are not limited thereto, and the number and location of the support protrusions 110b may be changed in various ways and applied. That is, one support protrusion 110b may also be provided at the center of the bottom surface of the seating groove 110a.

Moreover, the support protrusions 110b may also be formed as one body with the head portion 110, and may also be formed by applying a non-conductive material, for example, glue onto the bottom surface of the seating groove 110a.

### Industrial applicability

According to the present invention, a high-intensity focused ultrasound generation device, whereby vibration wave energy of a transducer may be maximized, can be manufactured.

## Claims

1. A structure for maximizing output of a vibration wave of a transducer holder of a high-intensity focused ultrasound generation device, the structure comprising:
an ultrasonic radiation frame having a concave front surface and a plurality of coupling holes formed therein;
a plurality of transducer holders respectively inserted into the plurality of coupling holes in front of the ultrasonic radiation frame and detachably coupled to the ultrasonic radiation frame while passing through the ultrasonic radiation frame; and
a plurality of transducers respectively mounted in the plurality of transducer holders so that front surfaces of the plurality of transducers are exposed,
wherein at least a part of a side surface of the transducer holder is incised to form an opening so as to maximize vibration wave energy generated in the transducer and radiating therefrom.

2. The structure of claim 1, wherein a flexible glue layer filled with flexible glue is formed between the transducer and the transducer holder.

3. The structure of claim 1, wherein each of the plurality of transducer holders comprises:
a head portion mounted on the front surface of the ultrasonic radiation frame, having a seating groove in which the transducer is insertedly seated, formed therein having the opening formed therein; and
a body portion extending from the head portion backward and coupled to the coupling holes using a fastening member from a rear of the ultrasonic radiation frame while passing through the coupling holes.

4. The structure of claim 3, wherein at least one support protrusion is protrudedly formed on a side surface of the seating groove to support a lower surface of the transducer and to form a separation space between the transducer and a bottom surface of the seating groove.

5. The structure of claim 3, wherein at least one support protrusion is protrudedly formed on the bottom surface of the seating groove to support a lower surface of the transducer and to form a separation space between the transducer and a bottom surface of the seating groove.

6. The structure of claim 4, wherein a flexible glue layer filled with flexible glue is formed between the lower surface of the transducer and an upper surface of the support protrusion to reduce loss of vibration wave energy of the transducer.

7. The structure of claim 5, wherein a flexible glue layer filled with flexible glue is formed between the lower surface of the transducer and the upper surface of the support protrusion to reduce loss of vibration wave energy of the transducer.

8. The structure of claim 3, wherein the body portion comprises power supply holes formed in a longitudinal direction so that a power supply unit coupled to the transducer passes through the power supply holes.

9. The structure of claim 7, wherein a glue layer for waterproofing filled with glue for waterproofing is formed between the power supply unit and the power supply holes.

10. The structure of claim 2, wherein the body portion comprises:
a shaft portion extending from the head portion backward and being pressed into the coupling holes; and
a screw portion extending from the shaft portion backward and coupled to the fastening member in the rear of the ultrasonic radiation frame after passing through the coupling holes.

11. A structure for maximizing output of a vibration wave of a transducer holder of a high-intensity focused ultrasound generation device, the structure comprising:
an ultrasonic radiation frame having a concave front surface and a plurality of coupling holes formed therein;
a plurality of transducer holders respectively inserted into the plurality of coupling holes in front of the ultrasonic radiation frame and detachably coupled to the ultrasonic radiation frame while passing through the ultrasonic radiation frame; and
a plurality of transducers respectively mounted in the plurality of transducer holders so that front surfaces of the plurality of transducers are exposed,
wherein the transducer holder comprises:
a head portion mounted on the front surface of the ultrasonic radiation frame and having a seating groove in which the transducer is insertedly seated, formed therein; and
a body portion extending from the head portion backward and coupled to the coupling holes using a fastening member from a rear of the ultrasonic radiation frame while passing through the coupling holes, and
at least a part of a side surface of the head portion is incised to form an opening so as to maximize vibration wave energy generated in the transducer, and
a support protrusion is protrudedly formed on a bottom surface of the seating groove to support a lower surface of the transducer and to form a separation space between the transducer and the bottom surface, and
a flexible glue layer filled with flexible glue is formed between the transducer and the support protrusion.
